# EUROPEAN PATENT SPECIFICATION

(11) **EP 3 846 684 B1**
(45) Date of publication and mention of the grant of the patent: **01.02.2023**
(21) Application number: 19836241.0
(22) Date of filing: 09.12.2019
(51) Int. Cl.: A61B 5/287

(54) **SYSTEM AND METHOD FOR MAPPING CARDIAC ACTIVATION WAVEFRONTS**
SYSTEM UND VERFAHREN ZUR KARTIERUNG KARDIALER AKTIVIERUNGSWELLENFRONTEN
SYSTÈME ET PROCÉDÉ POUR CARTOGRAPHIER DES FRONTS D'ONDE D'ACTIVATION CARDIAQUE

(30) Priority: 03.01.2019 US 201962787962 P
(43) Date of publication of application: 14.07.2021
(73) Proprietor: St. Jude Medical, Cardiology Division, Inc., St. Paul MN 55117 (US)
(72) Inventor: MANGUAL-SOTO, Jan O., 20017 Rho (IT); CALORE, Federico, 20134 Milan (IT)
(74) Representative: Kramer Barske Schmidtchen Patentanwälte PartG mbB
(86) International application number: PCT/US2019/065241
(87) International publication number: WO 2020/142165

(56) References cited:
- US-A1- 2017 055 864
- US-A1- 2018 042 503
- HOLM M ET AL: "A NEW METHOD FOR ANALYSIS OF ATRIAL ACTIVATION DURING CHRONIC ATRIAL FIBRILLATION IN MAN", IEEE TRANSACTIONS ON BIOMEDICAL ENGINEERING, IEEE SERVICE CENTER, PISCATAWAY, NJ, USA, vol. 43, no. 2, 1 February 1996 (1996-02-01), pages 198-210, XP000628427, ISSN: 0018-9294, DOI: 10.1109/10.481989
- G. LEE ET AL: "Epicardial wave mapping in human long-lasting persistent atrial fibrillation: transient rotational circuits, complex wavefronts, and disorganized activity", EUROPEAN HEART JOURNAL, vol. 35, no. 2, 8 August 2013 (2013-08-08) , pages 86-97, XP055372978, GB ISSN: 0195-668X, DOI: 10.1093/eurheartj/eht267

## Description

### CROSS-REFERENCE TO RELATED APPLICATIONS

This application claims the benefit of United States provisional application no. 62/787,962, filed 3 January 2019.

### BACKGROUND

The present disclosure relates generally to electrophysiological mapping, such as may be performed in cardiac diagnostic and therapeutic procedures. In particular, the present disclosure relates to systems, apparatuses, and methods for identifying and mapping cardiac activation wavefronts, as may be measured by multi-dimensional catheters such as high density ("HD") grid catheters.

Cardiac tissue is often characterized in connection with, *inter alia,* complex arrhythmia ablation procedures. For instance, a conduction velocity ("CV") map can be used to display the direction and speed of the electrical conduction at a given map point. One approach to computing a CV map is to gather local activation times ("LAT") of neighboring points.

It can also be of interest in an electrophysiology study to identify different wavefront patterns (*e.g.,* information regarding electrical propagation along the cardiac surface). Multiple wavefront patterns might occur during cardiac activations, including, for example, collision, focal, re-entry, and rotor. The identification and interpretation of these wavefronts can help analyze mechanistic properties of a broad range of electrophysiological pathologies. To study these wavefront patterns, however, one must first be able to identify them.

US 2018/0042503 A1 discloses a method for identifying rotors from bipolar EGM datasets recorded during cardiac fibrillation using a multipolar circular catheter. If a circular electrode array is placed over a rotor during cardiac fibrillation, the electrical wave will first be detected by one bipole, then the bipole immediately next to it, and so on until the wave returns to activate the first bipole again.

HOLM M ET AL: "A NEW METHOD FOR ANALYSIS OF ATRIAL ACTIVATION DURING CHRONIC ATRIAL FIBRILLATION IN MAN", IEEE TRANSACTIONS ON BIOMEDICAL ENGINEERING, IEEE SERVICE CENTER, PISCATAWAY, NJ, USA, vol. 43, no. 2, 1 February 1996 (1996-02-01), pages 198-210, XP000628427, ISSN: 0018-9294, DOI: 10.1109/10.481989 discloses an epicardial wave mapping in human long-lasting persistent atrial fibrillation.

G. LEE ET AL: "Epicardial wave mapping in human long-lasting persistent atrial fibrillation: transient rotational circuits, complex wavefronts, and disorganized activity", EUROPEAN HEART JOURNAL, vol. 35, no. 2, 8 August 2013 (2013-08-08), pages 86-97, XP055372978, GB ISSN: 0195-668X, DOI: 10.1093/eurheartj/eht267 discloses a method for analysing atrial activation during chronic atrial fibrillation by calculating the time difference between LAT's at adjacent measurement points into spatial dimensions.

### BRIEF SUMMARY

A method in accordance with the invention is defined in claim 1. An apparatus in accordance with the invention is defined in claim 5.

Disclosed herein is a method of mapping a cardiac activation wavefront as one or more stream paths. The method includes the steps of receiving, at an electroanatomical mapping system, electrophysiological data from a plurality of electrodes carried by a multi-electrode catheter, the plurality of electrodes defining a plurality of bipoles and the electrophysiological data including activation timing information; and the electroanatomical mapping system executing the steps of: identifying a first activating bipole of the plurality of bipoles from the activation timing information; and identifying an activation stream path, starting from the first activating bipole and ending at a last activating bipole, from the activation timing information.

The step of identifying an activation stream path, starting from the first activating bipole and ending at a last activating bipole, includes identifying an initial subset of the plurality of bipoles neighboring the first activating bipole; identifying an earliest activating bipole of the initial subset of the plurality of bipoles; adding the earliest activating bipole of the initial subset of the plurality of bipoles to the activation stream path as a next activating bipole; and repeating the steps of: identifying a subsequent subset of the plurality of bipoles neighboring the next activating bipole; identifying an earliest activating bipole of the subsequent subset of the plurality of bipoles; adding the earliest activating bipole of the subsequent subset of the plurality of bipoles to the activation stream path as the next activating bipole; until the last activating bipole is added to activation stream path.

Optionally, the method includes outputting a graphical representation of the activation stream path on a cardiac geometry model.

The method can also include the electroanatomical mapping system identifying an additional activation stream path by executing the steps of selecting a bipole of the plurality of bipoles that is not part of the activation stream path; and identifying the additional activation stream path, starting from the selected bipole, from the activation timing information.

In aspects of the disclosure, the method also includes repeating the steps of receiving, at an electroanatomical mapping system, electrophysiological data from a plurality of electrodes carried by a multi-electrode catheter, the plurality of electrodes defining a plurality of bipoles and the electrophysiological data including activation timing information; and the electroanatomical mapping system executing the steps of: identifying a first activating bipole of the plurality of bipoles from the activation timing information; and identifying an activation stream path, starting from the first activating bipole and ending at a last activating bipole, from the activation timing information for a plurality of locations of the multi-electrode catheter within a subject's heart.

Also disclosed herein is a method of mapping a cardiac activation wavefront as one or more stream paths. The method includes receiving, at an electroanatomical mapping system, electrophysiological data from a plurality of electrodes carried by a multi-electrode catheter, the electrophysiological data including activation timing information; and the electroanatomical mapping system executing the steps of: computing a conduction velocity vector field for the electrophysiological data over a plurality of cardiac geometry mapping points; defining at least one seed point from the plurality of cardiac geometry mapping points; and identifying at least one activation stream path for the at least one seed point from the computed conduction velocity vector field.

The step of computing a conduction velocity vector field for the electrophysiological data over a plurality of cardiac geometry mapping points can include computing the conduction velocity vector field over a plurality of cardiac geometry mapping points arranged in a uniform grid.

It is contemplated that the method can include outputting a graphical representation of the at least one activation stream path on a cardiac geometry model, wherein the cardiac geometry model is defined by the plurality of cardiac geometry mapping points. Optionally, the method can also include outputting a graphical representation of the conduction velocity vector field on the cardiac geometry model.

The instant disclosure also provides a method of mapping a cardiac activation wavefront as one or more stream paths. The method includes the steps of: receiving a geometry of at least a portion of a cardiac surface at an electroanatomical mapping system, the geometry comprising a plurality of nodes; receiving electrophysiology data for the portion of the cardiac surface at the electroanatomical mapping system; and the electroanatomical mapping system executing the steps of: defining a conduction velocity mesh; defining at least one seed point within the conduction velocity mesh; and identifying at least one activation stream path for the at least one seed point through the conduction velocity mesh.

According to aspects of the disclosure, the step of defining a conduction velocity mesh includes using the electrophysiology data to assign conduction velocity vectors across the geometry. The plurality of nodes can be arranged in a uniform grid.

The method can also include outputting a graphical representation of the at least one activation stream path for the at least one seed point and/or a graphical representation of the conduction velocity mesh on the geometry.

The disclosure also provides an electroanatomical mapping system for mapping cardiac activation wavefronts as one or more stream paths, including a stream path identification processor configured to: receive electrophysiological data, including activation timing information, from a plurality of electrodes carried by a multi-electrode catheter, the plurality of electrodes defining a plurality of bipoles; identify a first activating bipole of the plurality of bipoles from the activation timing information; and identify an activation stream path, starting from the first activating bipole and ending at a last activating bipole, from the activation timing information. The stream path identification processor can also be configured to identify the activation stream path by iteratively identifying successively activating bipoles of the plurality of bipoles from the activation timing information.

In embodiments, the system further includes a mapping processor configured to output a graphical representation of the activation stream path on a cardiac geometry model.

Also disclosed herein is an electroanatomical mapping system for mapping cardiac activation wavefronts as one or more stream paths, including a stream path identification processor configured to: receive electrophysiological data, including activation timing information, from a plurality of electrodes carried by a multi-electrode catheter; compute a conduction velocity vector field for the electrophysiological data over a plurality of cardiac geometry mapping points; and identify at least one activation stream path through the conduction velocity vector field. The stream path identification processor can be configured to compute the conduction velocity vector field over a uniformly distributed grid of geometry mapping points, and can be configured to identify at least one activation stream path through the conduction velocity vector field by identifying a path of at least one seed point through the conduction velocity vector field.

The system can also include a mapping processor configured to output a graphical representation of the at least one activation stream path on a cardiac geometry model.

The foregoing and other aspects, features, details, utilities, and advantages of the present invention will be apparent from reading the following description and claims, and from reviewing the accompanying drawings.

### BRIEF DESCRIPTION OF THE DRAWINGS

Figure 1 is a schematic diagram of an exemplary electroanatomical mapping system.
Figure 2 depicts an exemplary catheter that can be used in connection with aspects of the instant disclosure.
Figure 3 is a flowchart of representative steps that can be carried out according to exemplary embodiments disclosed herein.
Figures 4A and 4B provide alphanumeric labeling conventions for electrodes carried by a multi-electrode catheter and the bipoles associated therewith.
Figures 5A through 5D depict the identification of an activation stream path.
Figure 6 depicts a plurality of activation stream paths identified for a multi-electrode catheter.
Figure 7 depicts the creation of an activation stream path map for multiple positions of a multi-electrode catheter according to aspects of the instant teachings.
Figure 8 is a flowchart of representative steps that can be carried out according to additional embodiments disclosed herein.
Figure 9 depicts a geometric surface model including a plurality of nodes.
Figures 10A through 10D depict the creation of a streamline map from a conduction velocity mesh according to aspects of the instant teachings.

While multiple embodiments are disclosed, still other embodiments of the present disclosure will become apparent to those skilled in the art from the following detailed description, which shows and describes illustrative embodiments. Accordingly, the drawings and detailed description are to be regarded as illustrative in nature and not restrictive.

### DETAILED DESCRIPTION

The instant disclosure provides systems, apparatuses, and methods for mapping cardiac activation wavefronts and, more particularly, for mapping cardiac activation wavefronts as stream paths. For purposes of illustration, aspects of the disclosure will be described with reference to electrophysiological data collected using a high density (HD) grid catheter, such as the Advisor^{™} HD grid mapping catheter from Abbott Laboratories (Abbott Park, Illinois), in conjunction with an electroanatomical mapping system, such as the EnSite Precision^{™} cardiac mapping system, also from Abbott Laboratories. Those of ordinary skill in the art will understand, however, how to apply the teachings herein to good advantage in other contexts and/or with respect to other devices.

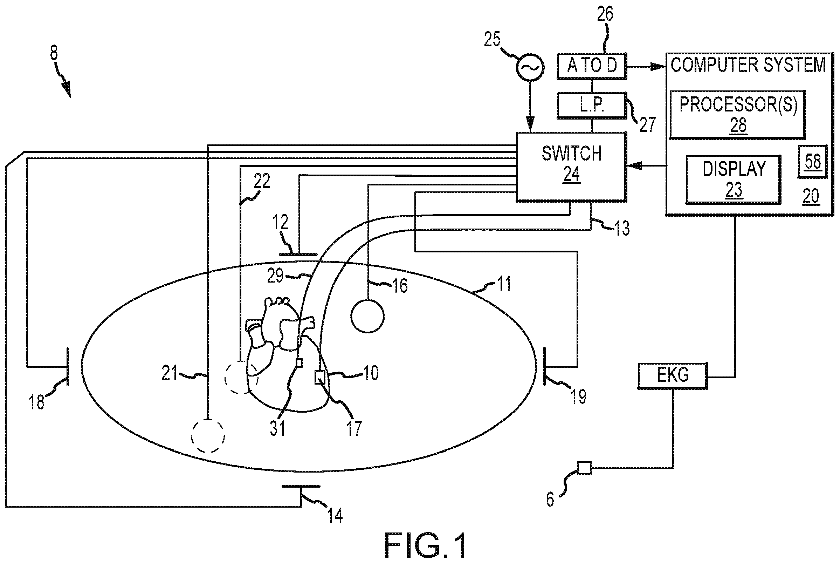

Figure 1 shows a schematic diagram of an exemplary electroanatomical mapping system 8 for conducting cardiac electrophysiology studies by navigating a cardiac catheter and measuring electrical activity occurring in a heart 10 of a patient 11 and three-dimensionally mapping the electrical activity and/or information related to or representative of the electrical activity so measured. System 8 can be used, for example, to create an anatomical model of the patient's heart 10 using one or more electrodes. System 8 can also be used to measure electrophysiology data at a plurality of points along a cardiac surface and store the measured data in association with location information for each measurement point at which the electrophysiology data was measured, for example to create a diagnostic data map of the patient's heart 10.

As one of ordinary skill in the art will recognize, system 8 determines the location, and in some aspects the orientation, of obj ects, typically within a three-dimensional space, and expresses those locations as position information determined relative to at least one reference.

For simplicity of illustration, the patient 11 is depicted schematically as an oval. In the embodiment shown in Figure 1, three sets of surface electrodes (e.g., patch electrodes) are shown applied to a surface of the patient 11, defining three generally orthogonal axes, referred to herein as an x-axis, a y-axis, and a z-axis. In other embodiments the electrodes could be positioned in other arrangements, for example multiple electrodes on a particular body surface. As a further alternative, the electrodes do not need to be on the body surface, but could be positioned internally to the body.

In Figure 1, the x-axis surface electrodes 12, 14 are applied to the patient along a first axis, such as on the lateral sides of the thorax region of the patient (e.g., applied to the patient's skin underneath each arm) and may be referred to as the Left and Right electrodes. The y-axis electrodes 18, 19 are applied to the patient along a second axis generally orthogonal to the x-axis, such as along the inner thigh and neck regions of the patient, and may be referred to as the Left Leg and Neck electrodes. The z-axis electrodes 16, 22 are applied along a third axis generally orthogonal to both the x-axis and the y-axis, such as along the sternum and spine of the patient in the thorax region, and may be referred to as the Chest and Back electrodes. The heart 10 lies between these pairs of surface electrodes 12/14, 18/19, and 16/22.

An additional surface reference electrode (*e.g.,* a "belly patch") 21 provides a reference and/or ground electrode for the system 8. The belly patch electrode 21 may be an alternative to a fixed intra-cardiac electrode 31, described in further detail below. It should also be appreciated that, in addition, the patient 11 may have most or all of the conventional electrocardiogram ("ECG" or "EKG") system leads in place. In certain embodiments, for example, a standard set of 12 ECG leads may be utilized for sensing electrocardiograms on the patient's heart 10. This ECG information is available to the system 8 (*e.g.,* it can be provided as input to computer system 20). Insofar as ECG leads are well understood, and for the sake of clarity in the figures, only a single lead 6 and its connection to computer 20 is illustrated in Figure 1.

A representative catheter 13 having at least one electrode 17 is also shown. This representative catheter electrode 17 is referred to as the "roving electrode," "moving electrode," or "measurement electrode" throughout the specification. Typically, multiple electrodes 17 on catheter 13, or on multiple such catheters, will be used. In one embodiment, for example, the system 8 may comprise sixty-four electrodes on twelve catheters disposed within the heart and/or vasculature of the patient. In other embodiments, system 8 may utilize a single catheter that includes multiple (*e.g.,* eight) splines, each of which in turn includes multiple (*e.g.,* eight) electrodes.

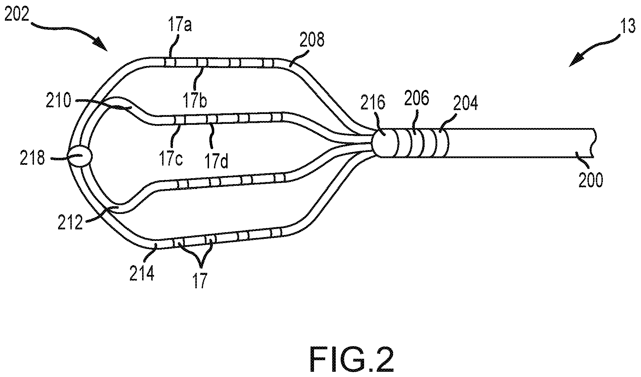

The foregoing embodiments are merely exemplary, however, and any number of electrodes and/or catheters may be used. For example, for purposes of this disclosure, a segment of an exemplary multi-electrode catheter, and in particular an HD grid catheter, is shown in Figure 2. HD grid catheter 13 includes a catheter body 200 coupled to a paddle 202. Catheter body 200 can further include first and second body electrodes 204, 206, respectively. Paddle 202 can include a first spline 208, a second spline 210, a third spline 212, and a fourth spline 214, which are coupled to catheter body 200 by a proximal coupler 216 and to each other by a distal coupler 218. In one embodiment, first spline 208 and fourth spline 214 can be one continuous segment and second spline 210 and third spline 212 can be another continuous segment. In other embodiments, the various splines 208, 210, 212, 214 can be separate segments coupled to each other (e.g., by proximal and distal couplers 216, 218, respectively). It should be understood that HD catheter 13 can include any number of splines; the four-spline arrangement shown in Figure 2 is merely exemplary.

As described above, splines 208, 210, 212, 214 can include any number of electrodes 17; in Figure 2, sixteen electrodes 17 are shown arranged in a four-by-four array. It should also be understood that electrodes 17 can be evenly and/or unevenly spaced, as measured both along and between splines 208, 210, 212, 214.

Catheter 13 (or multiple such catheters) are typically introduced into the heart and/or vasculature of the patient via one or more introducers and using familiar procedures. Indeed, various approaches to introduce catheter 13 into a patient's heart, such as transseptal approaches, will be familiar to those of ordinary skill in the art, and therefore need not be further described herein.

Since each electrode 17 lies within the patient, location data may be collected simultaneously for each electrode 17 by system 8. Similarly, each electrode 17 can be used to gather electrophysiological data from the cardiac surface (*e.g.,* surface electrograms). The ordinarily skilled artisan will be familiar with various modalities for the acquisition and processing of electrophysiology data points (including, for example, both contact and noncontact electrophysiological mapping), such that further discussion thereof is not necessary to the understanding of the techniques disclosed herein. Likewise, various techniques familiar in the art can be used to generate a graphical representation of a cardiac geometry and/or of cardiac electrical activity from the plurality of electrophysiology data points. Moreover, insofar as the ordinarily skilled artisan will appreciate how to create electrophysiology maps from electrophysiology data points, the aspects thereof will only be described herein to the extent necessary to understand the present disclosure.

Returning now to Figure 1, in some embodiments, an optional fixed reference electrode 31 (*e.g.,* attached to a wall of the heart 10) is shown on a second catheter 29. For calibration purposes, this electrode 31 may be stationary (*e.g.,* attached to or near the wall of the heart) or disposed in a fixed spatial relationship with the roving electrodes (*e.g.,* electrodes 17), and thus may be referred to as a "navigational reference" or "local reference." The fixed reference electrode 31 may be used in addition or alternatively to the surface reference electrode 21 described above. In many instances, a coronary sinus electrode or other fixed electrode in the heart 10 can be used as a reference for measuring voltages and displacements; that is, as described below, fixed reference electrode 31 may define the origin of a coordinate system.

Each surface electrode is coupled to a multiplex switch 24, and the pairs of surface electrodes are selected by software running on a computer 20, which couples the surface electrodes to a signal generator 25. Alternately, switch 24 may be eliminated and multiple (*e.g.,* three) instances of signal generator 25 may be provided, one for each measurement axis (that is, each surface electrode pairing).

The computer 20 may comprise, for example, a conventional general-purpose computer, a special-purpose computer, a distributed computer, or any other type of computer. The computer 20 may comprise one or more processors 28, such as a single central processing unit ("CPU"), or a plurality of processing units, commonly referred to as a parallel processing environment, which may execute instructions to practice the various aspects described herein.

Generally, three nominally orthogonal electric fields are generated by a series of driven and sensed electric dipoles (*e.g.,* surface electrode pairs 12/14, 18/19, and 16/22) in order to realize catheter navigation in a biological conductor. Alternatively, these orthogonal fields can be decomposed and any pairs of surface electrodes can be driven as dipoles to provide effective electrode triangulation. Likewise, the electrodes 12, 14, 18, 19, 16, and 22 (or any number of electrodes) could be positioned in any other effective arrangement for driving a current to or sensing a current from an electrode in the heart. For example, multiple electrodes could be placed on the back, sides, and/or belly of patient 11. Additionally, such non-orthogonal methodologies add to the flexibility of the system. For any desired axis, the potentials measured across the roving electrodes resulting from a predetermined set of drive (source-sink) configurations may be combined algebraically to yield the same effective potential as would be obtained by simply driving a uniform current along the orthogonal axes.

Thus, any two of the surface electrodes 12, 14, 16, 18, 19, 22 may be selected as a dipole source and drain with respect to a ground reference, such as belly patch 21, while the unexcited electrodes measure voltage with respect to the ground reference. The roving electrodes 17 placed in the heart 10 are exposed to the field from a current pulse and are measured with respect to ground, such as belly patch 21. In practice the catheters within the heart 10 may contain more or fewer electrodes than the sixteen shown, and each electrode potential may be measured. As previously noted, at least one electrode may be fixed to the interior surface of the heart to form a fixed reference electrode 31, which is also measured with respect to ground, such as belly patch 21, and which may be defined as the origin of the coordinate system relative to which system 8 measures positions. Data sets from each of the surface electrodes, the internal electrodes, and the virtual electrodes may all be used to determine the location of the roving electrodes 17 within heart 10.

The measured voltages may be used by system 8 to determine the location in three-dimensional space of the electrodes inside the heart, such as roving electrodes 17 relative to a reference location, such as reference electrode 31. That is, the voltages measured at reference electrode 31 may be used to define the origin of a coordinate system, while the voltages measured at roving electrodes 17 may be used to express the location of roving electrodes 17 relative to the origin. In some embodiments, the coordinate system is a three-dimensional (x, y, z) Cartesian coordinate system, although other coordinate systems, such as polar, spherical, and cylindrical coordinate systems, are contemplated.

As should be clear from the foregoing discussion, the data used to determine the location of the electrode(s) within the heart is measured while the surface electrode pairs impress an electric field on the heart. The electrode data may also be used to create a respiration compensation value used to improve the raw location data for the electrode locations as described, for example, in United States Patent No. 7,263,937. The electrode data may also be used to compensate for changes in the impedance of the body of the patient as described, for example, in United States Patent No. 7,885,707.

Therefore, in one representative embodiment, system 8 first selects a set of surface electrodes and then drives them with current pulses. While the current pulses are being delivered, electrical activity, such as the voltages measured with at least one of the remaining surface electrodes and in vivo electrodes, is measured and stored. Compensation for artifacts, such as respiration and/or impedance shifting, may be performed as indicated above.

In some embodiments, system 8 is the EnSite^{™} Velocity^{™} or EnSite Precision^{™} cardiac mapping and visualization system of Abbott Laboratories. Other localization systems, however, may be used in connection with the present teachings, including for example the RHYTHMIA HDX^{™} mapping system of Boston Scientific Corporation (Marlborough, Massachusetts), the CARTO navigation and location system of Biosense Webster, Inc. (Irvine, California), the AURORA^{®} system of Northern Digital Inc. (Waterloo, Ontario), Sterotaxis, Inc.'s NIOBE^{®} Magnetic Navigation System (St. Louis, Missouri), as well as MediGuide^{™} Technology from Abbott Laboratories.

The localization and mapping systems described in the following patents can also be used with the present invention: United States Patent Nos. 6,990,370; 6,978,168; 6,947,785; 6,939,309; 6,728,562; 6,640,119; 5,983,126; and 5,697,377.

Aspects of the disclosure relate to mapping cardiac activation wavefronts as one or more static stream paths; graphical representations of the stream paths (and other electrophysiological data) can also be output, for example on display 23. System 8 can therefore include a stream path identification module 58 that can be used to identify stream paths, and which may incorporate a mapping module to allow for graphical output thereof (*e.g.,* to display 23).

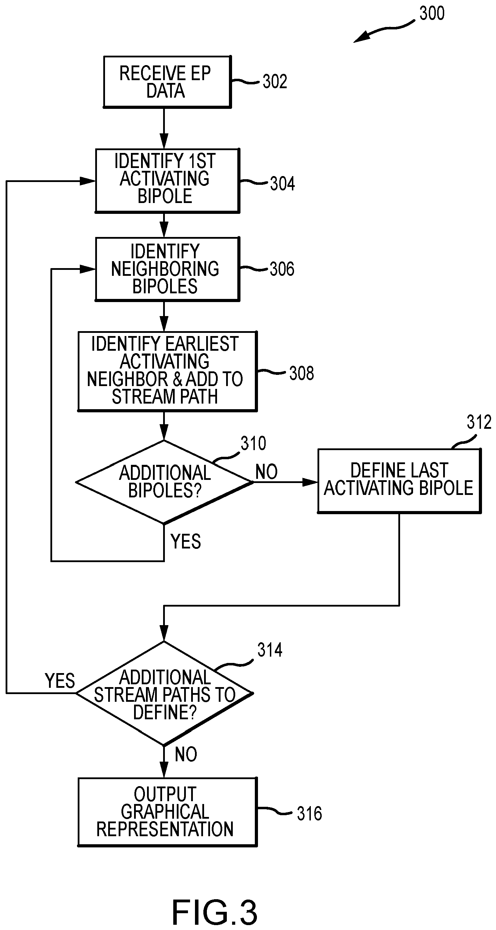

One exemplary method according to the present teachings will be explained with reference to the flowchart 300 of representative steps presented as Figure 3. In some embodiments, for example, flowchart 300 may represent several exemplary steps that can be carried out by electroanatomical mapping system 8 of Figure 1 (*e.g.,* by processor 28 and/or stream path identification module 58). It should be understood that the representative steps described below can be either hardware- or software-implemented. For the sake of explanation, the term "signal processor" is used herein to describe both hardware- and software-based implementations of the teachings herein.

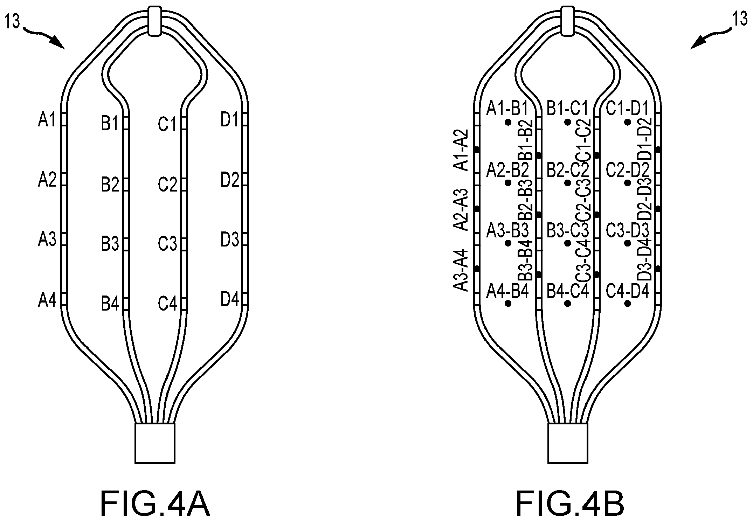

In block 302, system 8 receives electrophysiological data from electrodes 17 carried by catheter 13. For purposes of easy reference in this description, Figure 4A provides alphanumeric labels for electrodes 17.

As those of ordinary skill in the art will recognize, any two neighboring electrodes 17 define a bipole. Thus, the 16 electrodes 17 on catheter 13 define a total of 42 bipoles - 12 along splines (e.g., between electrodes 17a and 17b, or between electrodes 17c and 17d), 12 across splines (*e.g.,* between electrodes 17a and 17c, or between electrodes 17b and 17d), and 18 diagonally between splines (*e.g.,* between electrodes 17a and 17d, or between electrodes 17b and 17c).

For ease of reference in this description, Figure 4B provides alphanumeric labels for the along- and across-spline bipoles. Figure 4B omits alphanumeric labels for the diagonal bipoles, but this is only for the sake of clarity in the illustration. It is expressly contemplated that the teachings herein can also be applied with respect to the diagonal bipoles.

Any bipole can, in turn, be used to generate a bipolar electrogram, including activation timing information for the given bipole, according to techniques that will be familiar to those of ordinary skill in the art. Moreover, these bipolar electrograms can be combined (*e.g.,* linearly combined) to generate electrograms, again including activation timing information, in any direction of the plane of catheter 13 by computing an E-field loop for a clique of electrodes. United States application no. 15/953,155 discloses details of computing an E-field loop for a clique of electrodes on a HD grid catheter.

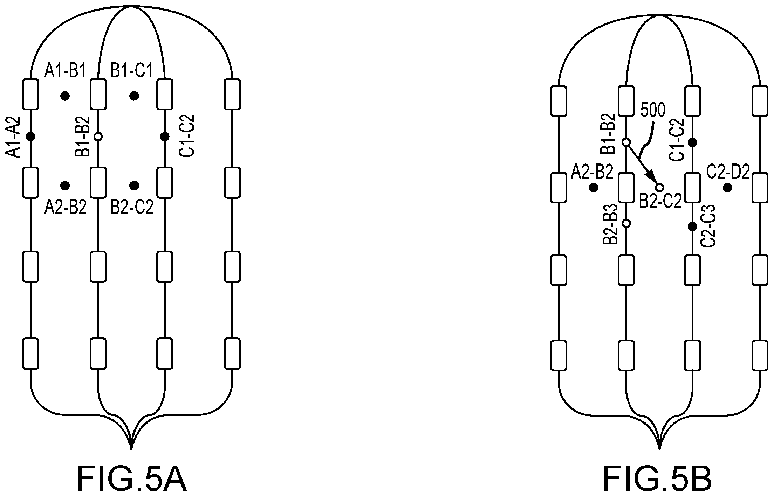

In block 304, the activation timing information for the plurality of bipoles is used to identify the first activating bipole (that is, the bipole with the earliest activation timing). For purposes of illustration, assume that the first activating bipole is bipole B1-B2, as shown in Figure 5A.

Blocks 306-312 represent a series of steps by which system 8 can identify an activation stream path, starting from the first activating bipole identified in block 304 and ending at a last activating bipole. In general, blocks 306-312 represent iteratively identifying successively activating, neighboring bipoles.

Thus, in block 306, system 8 identifies a first subset of the plurality of bipoles neighboring the first activating bipole identified in block 304. In the illustrative case of Figure 5A, this neighborhood includes bipoles A1-B1, B1-C1, A1-A2, C1-C2, A2-B2, and B2-C2.

In block 308, system 8 identifies the earliest activating bipole of the neighboring bipoles and adds that bipole to the activation stream path. For instance, in the illustrative case of Figure 5B, the earliest activating neighboring bipole is bipole B2-C2, and the activation stream path is represented by arrow 500.

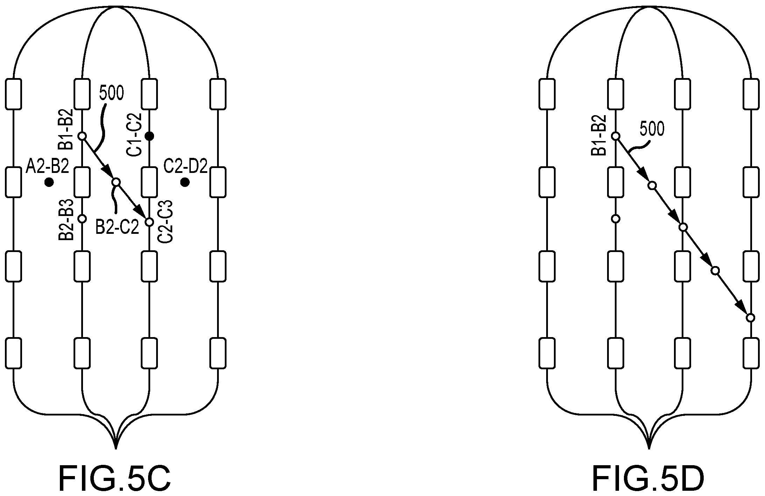

Decision block 310 considers whether there are additional, later-activating, bipoles neighboring the bipole most recently added to the activation stream path. If so, the process returns to blocks 306 and 308, to continue adding later-activating bipoles to the activation stream path. For instance, Figure 5C illustrates that, among bipoles C1-C2, A2-B2, C2-D2, B2-B3, and C2-C3 neighboring bipole B2-C2, bipole C2-C3 is the earliest activating. Thus, after one additional iteration through blocks 306 and 308, the activation stream path represented by arrows 500 further extends from bipole B2-C2 to bipole C2-C3. Figure 5D illustrates the result after two more iterations through blocks 306 and 308, adding bipoles C3-D3 and D3-D4 to the activation stream path represented by arrows 500.

Once bipole D3-D4 is added to the activation stream path, however, no additional, later-activating, neighboring bipoles are found. Thus, in block 312, bipole D3-D4 is defined as the last activating bipole, and the activation stream path beginning with bipole B1-B2 is complete.

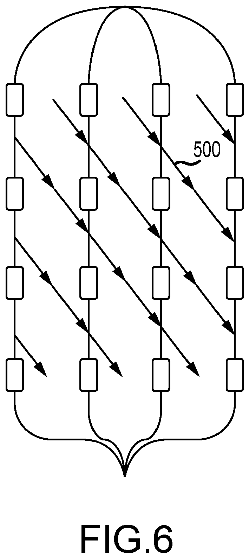

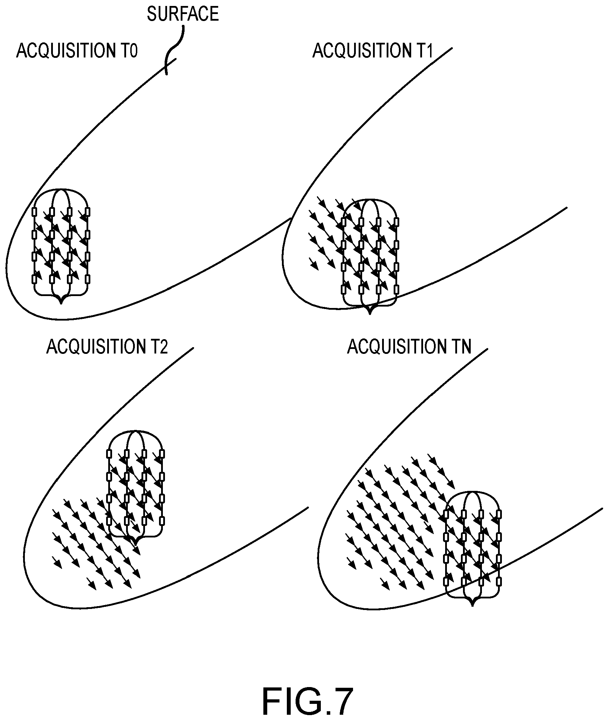

Decision block 314 examines whether there are additional stream paths to define, in which case the process loops back to block 304 to identify an activation stream path that starts at a new first activating bipole. Advantageously, additional stream paths can be defined starting from additional bipoles, for a given location in the subject's heart, as shown in Figure 6. Additional stream paths can also be defined for additional positions of catheter 13 within the subject's heart; for instance, Figure 7 depicts stream paths for four different positions of catheter 13 relative to a cardiac surface geometry. Indeed, in block 316, system 8 can output (*e.g.,* on display 23) a graphical representation of any identified stream paths on a cardiac geometry model.

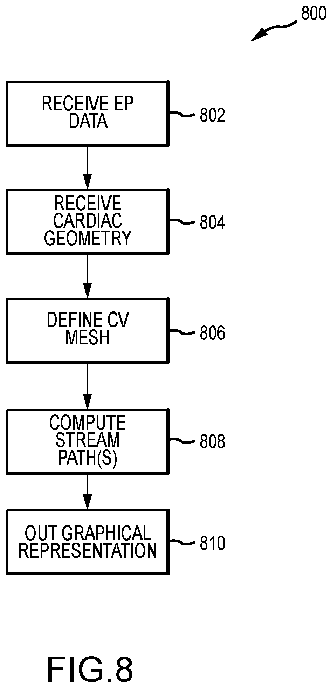

Another exemplary method according to the present teachings will be explained with reference to the flowchart 800 of representative steps presented as Figure 8. In some embodiments, for example, flowchart 800 may represent several exemplary steps that can be carried out by electroanatomical mapping system 8 of Figure 1 (*e.g.,* by processor 28 and/or stream path identification module 58). It should be understood that the representative steps described below can be either hardware- or software-implemented. For the sake of explanation, the term "signal processor" is used herein to describe both hardware- and software-based implementations of the teachings herein.

In block 802, system 8 receives electrophysiological data from electrodes 17 carried by catheter 13. Such electrophysiological data is described above in connection with Figure 3.

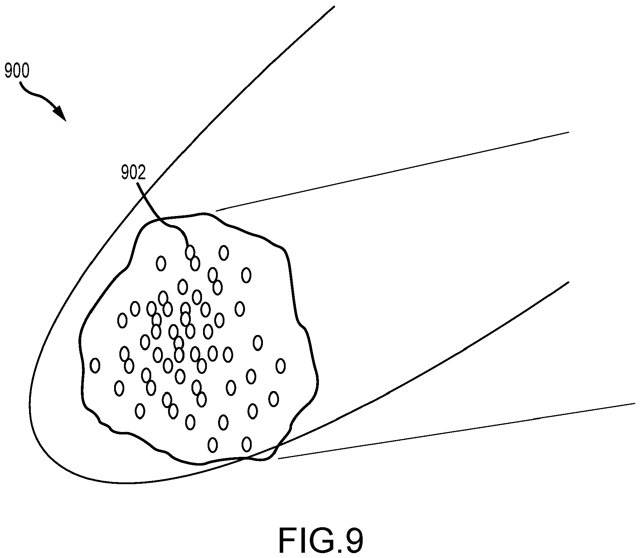

In block 804, system 8 receives a geometry of at least a portion of a cardiac surface. Figure 9 depicts a representative three-dimensional surface geometry 900, which includes a plurality of nodes 902 (nodes 902 can also be referred to as "geometry mapping points" or, more simply, "geometry points").

In block 806, system 8 computes a conduction velocity mesh for the portion of the cardiac surface. As used herein, the term "conduction velocity mesh" refers to a conduction velocity vector field over the plurality of nodes 902 of geometry 900 (that is, a plurality of geometry mapping points with conduction velocity vectors assigned thereto).

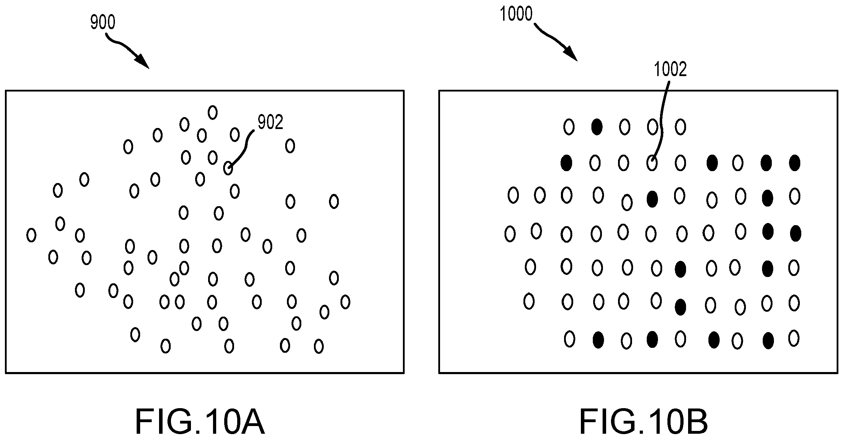

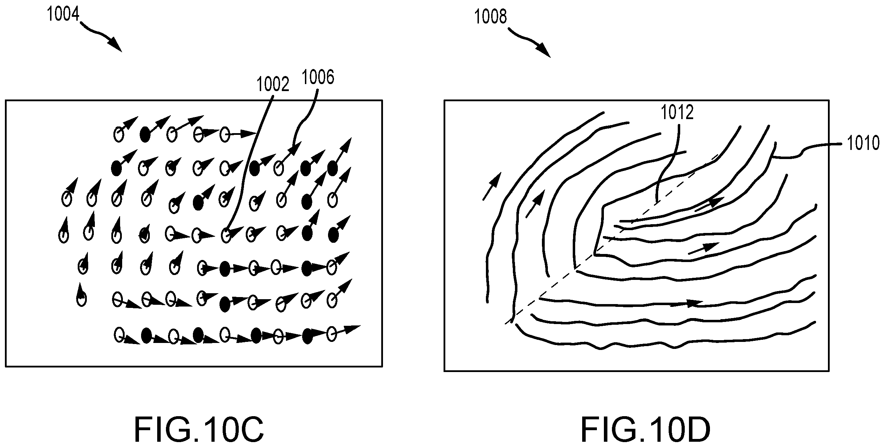

In embodiments of the instant disclosure, the conduction velocity mesh includes a substantially uniform grid of nodes. For instance, Figure 10A depicts original nodes 902 of geometry 900, while Figure 10B depicts a substantially uniform grid 1000 of nodes 1002. Figure 10C, in turn, depicts the conduction velocity mesh 1004 (e.g., substantially uniformly distributed nodes 1002 with conduction velocity vectors 1006 assigned thereto). Those of ordinary skill in the art will be familiar with various techniques useful in the creation of a conduction velocity mesh as described herein, although one approach thereto is described in United States patent no. 9,888,860.

In block 808, system 8 computes at least one activation stream path through the conduction velocity mesh. More particularly, each activation stream path is computed for a corresponding seed point; the seed points may be randomly generated by system 8, user selected, or a combination thereof. The path of each seed point through the conduction velocity vector field is analyzed in a manner analogous to streamline calculations in fluid flow analysis (*e.g.,* using a vector field to calculate the stream path of individual spatial points).

In block 810, a graphical representation of the activation stream path(s) can be output (*e.g.,* on display 23) on a model of the cardiac surface. Figure 10D depicts, in two dimensions, an activation stream path map 1008 including a plurality of streamlines 1010. Optionally, system 8 can also output a graphical representation of the conduction velocity mesh (*e.g.,* as shown in Figure 10C).

Stream path lines 500 (as shown in Figure 5A through 7) and/or streamlines 1010 (as shown in Figure 10D) advantageously allow a practitioner to rapidly identify regions of conduction divergence (*e.g.,* focal source or region of block) and/or convergence (*e.g.,* regions of colliding wavefront). For instance, in Figure 10D, line 1012 represents a line of divergence.

Although several embodiments have been described above with a certain degree of particularity, those skilled in the art could make numerous alterations to the disclosed embodiments without departing from the scope of this invention, which is defined in the claims.

For example, the teachings herein can be applied in real time (*e.g.,* during an electrophysiology study) or during post-processing (*e.g.,* to electrophysiology data points collected during an electrophysiology study performed at an earlier time).

All directional references (*e.g.,* upper, lower, upward, downward, left, right, leftward, rightward, top, bottom, above, below, vertical, horizontal, clockwise, and counterclockwise) are only used for identification purposes to aid the reader's understanding of the present invention, and do not create limitations, particularly as to the position, orientation, or use of the invention. Joinder references (*e.g.,* attached, coupled, connected, and the like) are to be construed broadly and may include intermediate members between a connection of elements and relative movement between elements. As such, joinder references do not necessarily infer that two elements are directly connected and in fixed relation to each other.

It is intended that all matter contained in the above description or shown in the accompanying drawings shall be interpreted as illustrative only and not limiting. Changes in detail or structure may be made without departing from the scope of the invention as defined in the appended claims.

## Claims

1. A method of mapping a cardiac activation wavefront as one or more stream paths, comprising:
receiving (302), at an electroanatomical mapping system, electrophysiological data from a plurality of electrodes (17) carried by a multi-electrode catheter (13), the plurality of electrodes defining a plurality of bipoles and the electrophysiological data including activation timing information; and
the electroanatomical mapping system executing the steps of:
identifying (304) a first activating bipole of the plurality of bipoles from the activation timing information; and
identifying an activation stream path, starting from the first activating bipole and ending at a last activating bipole, from the activation timing information,
wherein identifying an activation stream path, starting from the first activating bipole and ending at a last activating bipole, comprises:
identifying (306) an initial subset of the plurality of bipoles neighboring the first activating bipole;
identifying (308) an earliest activating bipole of the initial subset of the plurality of bipoles;
adding (310) the earliest activating bipole of the initial subset of the plurality of bipoles to the activation stream path as a next activating bipole; and
repeating the steps of:
identifying (306) a subsequent subset of the plurality of bipoles neighboring the next activating bipole;
identifying (308) an earliest activating bipole of the subsequent subset of the plurality of bipoles;
adding (310) the earliest activating bipole of the subsequent subset of the plurality of bipoles to the activation stream path as the next activating bipole; until the last activating bipole is added to activation stream path.

2. The method according to claim 1, further comprising outputting (316) a graphical representation of the activation stream path on a cardiac geometry model.

3. The method according to claim 1 or 2, further comprising the electroanatomical mapping system identifying (314) an additional activation stream path by executing the steps of:
selecting a bipole of the plurality of bipoles that is not part of the activation stream path; and
identifying the additional activation stream path, starting from the selected bipole, from the activation timing information.

4. An electroanatomical mapping system for mapping cardiac activation wavefronts as one or more stream paths, comprising:
a stream path identification processor (28) configured to:
receive electrophysiological data, including activation timing information, from a plurality of electrodes (17) carried by a multi-electrode catheter (13), the plurality of electrodes defining a plurality of bipoles;
identify a first activating bipole of the plurality of bipoles from the activation timing information; and
identify an activation stream path, starting from the first activating bipole and ending at a last activating bipole, from the activation timing information,
wherein identifying an activation stream path, starting from the first activating bipole and ending at a last activating bipole, comprises:
identifying (306) an initial subset of the plurality of bipoles neighboring the first activating bipole;
identifying (308) an earliest activating bipole of the initial subset of the plurality of bipoles; adding (310) the earliest activating bipole of the initial subset of the plurality of bipoles to the activation stream path as a next activating bipole;
the stream path identification processor (28) being further configured to repeat:
identifying (306) a subsequent subset of the plurality of bipoles neighboring the next activating bipole;
identifying (308) an earliest activating bipole of the subsequent subset of the plurality of bipoles; and
adding (310) the earliest activating bipole of the subsequent subset of the plurality of bipoles to the activation stream path as the next activating bipole;
until the last activating bipole is added to activation stream path,
wherein the stream path identification processor is configured to identify the activation stream path by iteratively identifying successively activating bipoles of the plurality of bipoles from the activation timing information.

5. The system according to claim 4 , further comprising a mapping processor configured to output a graphical representation of the activation stream path on a cardiac geometry model.

## Patentansprüche

1. Verfahren zum Abbilden einer Herzaktivierungswellenfront als einen oder als mehrere Strompfade, mit:
Empfangen (302), an einem elektroanatomischen Abbildungssystem, von elektrophysiologischen Daten von einer Mehrzahl von Elektroden (17), die von einem Mehrelektrodenkatheter (13) getragen werden, wobei die Mehrzahl der Elektroden eine Mehrzahl von Bipolen definiert, und die elektrophysiologischen Daten eine Aktivierungszeitinformation aufweisen; und
das elektroanatomische Abbildungssystem die Schritte ausführt:
Identifizieren (304) eines ersten Aktivierungsbipols von der Mehrzahl von Bipolen anhand der Aktivierungszeitinformation; und
Identifizieren eines Aktivierungsstrompfads, der bei dem ersten Aktivierungsbipol beginnt und bei einem letzten Aktivierungsbipol endet, anhand der Aktivierungszeitinformation, wobei
das Identifizieren eines Aktivierungsstrompfads, der von dem ersten Aktivierungsbipol beginnt und bei einem letzten Aktivierungsbipol endet, aufweist:
Identifizieren (306) eines anfänglichen Nebensatzes von der Mehrzahl von Bipolen, die benachbart zu dem ersten Aktivierungsbipol sind;
Identifizieren (308) eines frühesten Aktivierungsbipols von dem anfänglichen Nebensatz von der Mehrzahl von Bipolen;
Hinzufügen (310) des frühesten Aktivierungsbipols von dem anfänglichen Nebensatz von der Mehrzahl von Bipolen zu dem Aktivierungsstrompfad als einen nächsten Aktivierungsbipol; und
Wiederholen der Schritte:
Identifizieren (306) eines nachfolgenden Nebensatzes von der Mehrzahl von Bipolen, die benachbart sind zu dem nächsten Aktivierungsbipol;
Identifizieren (308) eines frühesten Aktivierungsbipols von dem nachfolgenden Nebensatz von der Mehrzahl von Bipolen;
Hinzufügen (310) des frühesten Aktivierungsbipols von dem nachfolgenden Nebensatz von der Mehrzahl von Bipolen zu dem Aktivierungsstrompfad als nächsten Aktivierungsbipol;
bis der letzte Aktivierungsbipol zu dem Aktivierungsstrompfad hinzugefügt ist.

2. Verfahren nach Anspruch 1, ferner mit einem Ausgeben (316) einer grafischen Darstellung des Aktivierungsstrompfads auf einem Herzgeometriemodel.

3. Verfahren nach Anspruch 1 oder 2, bei dem das elektroanatomische Abbildungssystem ferner einen zusätzlichen Aktivierungsstrompfad identifiziert (314), indem die Schritte durchgeführt werden:
Auswählen eines Bipols von der Mehrzahl von Bipolen, der nicht Teil des Aktivierungsstrompfads ist; und
Identifizieren des zusätzlichen Aktivierungsstrompfads, der bei dem ausgewählten Bipol beginnt, anhand der Aktivierungszeitinformation.

4. Elektroanatomisches Abbildungssystem zum Abbilden von Herzaktivierungswellenfronten als einen oder als mehrere Strompfade, mit:
einem Strompfadidentifikationsprozessor (28), der konfiguriert ist zum:
Empfangen von elektrophysiologischen Daten, die Aktivierungszeitinformation aufweisen, von einer Mehrzahl von Elektroden (17), die von einem Mehrelektrodenkatheter (13) getragen werden, wobei die Mehrzahl der Elektroden eine Mehrzahl von Bipolen definiert;
Identifizieren eines ersten Aktivierungsbipols von der Mehrzahl von Bipolen anhand der Aktivierungszeitinformation; und
Identifizieren eines Aktivierungsstrompfads, der bei dem ersten Aktivierungsbipol beginnt und bei einem letzten Aktivierungsbipol endet, anhand der Aktivierungszeitinformation, wobei
das Identifizieren eines Aktivierungsstrompfads, der bei dem ersten Aktivierungsbipol beginnt und bei einem letzten Aktivierungsbipol endet, aufweist:
Identifizieren (306) eines anfänglichen Nebensatzes von der Mehrzahl von Bipolen, die benachbart zu dem ersten Aktivierungsbipol sind;
Identifizieren (308) eines frühesten Aktivierungsbipols von dem anfänglichen Nebensatz von der Mehrzahl von Bipolen;
Hinzufügen (310) des frühesten Aktivierungsbipols von dem anfänglichen Nebensatz von der Mehrzahl von Bipolen zu dem Aktivierungsstrompfad als nächsten Aktivierungsbipol;
der Strompfadidentifikationsprozessor (28) ferner konfiguriert ist zum Wiederholen von:
dem Identifizieren (306) eines nachfolgenden Nebensatzes von der Mehrzahl von Bipolen, die benachbart zu dem nächsten Aktivierungsbipol sind;
dem Identifizieren (308) eines frühesten Aktivierungsbipols von dem nachfolgenden Nebensatz von der Mehrzahl von Bipolen; und
dem Hinzufügen (310) des frühesten Aktivierungsbipols von dem nachfolgenden Nebensatz von der Mehrzahl von Bipolen zu dem Aktivierungsstrompfad als nächsten Aktivierungsbipol;
bis der letzte Aktivierungsbipol zu dem Aktivierungsstrompfad hinzugefügt ist, wobei
der Strompfadidentifikationsprozessor konfiguriert ist zum Identifizieren des Aktivierungsstrompfads durch iteratives Identifizieren nachfolgender Aktivierungsbipole von der Mehrzahl von Bipolen anhand der Aktivierungszeitinformation.

5. System nach Anspruch 4, ferner mit einem Abbildungsprozessor, der konfiguriert ist zum Ausgeben einer grafischen Darstellung des Aktivierungsstrompfads auf einem Herzgeometriemodel.

## Revendications

1. Procédé de cartographie d'un front d'onde d'activation cardiaque sous la forme d'un ou plusieurs trajets de flux, comprenant les étapes consistant à :
recevoir (302), au niveau d'un système de cartographie électro-anatomique, de données électrophysiologiques provenant d'une pluralité d'électrodes (17) portées par un cathéter multi-électrodes (13), la pluralité d'électrodes définissant une pluralité de bipôles et les données électrophysiologiques comprenant des informations de synchronisation d'activation ; et
le système de cartographie électro-anatomique exécutant les étapes consistant à :
identifier (304) un premier bipôle d'activation de la pluralité de bipôles à partir des informations de synchronisation d'activation ; et
identifier un trajet de flux d'activation, commençant à partir du premier bipôle d'activation et se terminant à un dernier bipôle d'activation, à partir des informations de synchronisation d'activation,
dans lequel l'identification d'un trajet de flux d'activation, commençant à partir du premier bipôle d'activation et se terminant à un dernier bipôle d'activation, comprend les étapes consistant à :
identifier (306) un sous-ensemble initial de la pluralité de bipôles voisins du premier bipôle d'activation ;
identifier (308) un bipôle d'activation le plus précoce du sous-ensemble initial de la pluralité de bipôles ;
ajouter (310) le bipôle d'activation le plus précoce du sous-ensemble initial de la pluralité de bipôles au trajet de flux d'activation en tant que bipôle d'activation suivant ; et
répéter les étapes consistant à :
identifier (306) un sous-ensemble subséquent de la pluralité de bipôles voisin du bipôle d'activation suivant ;
identifier (308) un bipôle d'activation le plus précoce du sous-ensemble subséquent de la pluralité de bipôles ;
ajouter (310) le bipôle d'activation le plus précoce du sous-ensemble subséquent de la pluralité de bipôles au trajet de flux d'activation en tant que bipôle d'activation suivant ;
jusqu'à ce que le dernier bipôle d'activation soit ajouté au trajet de flux d'activation.

2. Procédé selon la revendication 1, comprenant en outre la sortie (316) d'une représentation graphique du trajet de flux d'activation sur un modèle géométrique cardiaque.

3. Procédé selon la revendication 1 ou 2, comprenant en outre l'identification (314) par le système de cartographie électro-anatomique d'un trajet de flux d'activation supplémentaire en exécutant les étapes consistant à :
sélectionner un bipôle de la pluralité de bipôles qui ne fait pas partie du trajet de flux d'activation ; et
identifier le trajet de flux d'activation supplémentaire, en commençant par le bipôle sélectionné, à partir des informations de synchronisation d'activation.

4. Système de cartographie électro-anatomique pour cartographier des fronts d'onde d'activation cardiaque en tant qu'un ou plusieurs trajets de flux, comprenant :
un processeur d'identification de trajet de flux (28) configuré pour :
recevoir des données électrophysiologiques, y compris des informations de synchronisation d'activation, devant une pluralité d'électrodes (17) portées par un cathéter multi-électrodes (13), la pluralité d'électrodes déduisant une pluralité de bipôles ;
identifier un premier bipôle d'activation de la pluralité de bipôles devant les informations de synchronisation d'activation ; et
identifier un trajet de flux d'activation, commençant par le premier bipôle d'activation et se terminant à un dernier bipôle d'activation, devant les informations de synchronisation d'activation,
dans lequel l'identification d'un trajet de flux d'activation, commençant par le premier bipôle d'activation et se terminant à un dernier bipôle d'activation, comprend les étapes consistant à :
identifier (306) un sous-ensemble initial de la pluralité de bipôles voisins du premier bipôle d'activation ;
identifier (308) un bipôle d'activation le plus précoce du sous-ensemble initial de la pluralité de bipôles ;
ajouter (310) le bipôle d'activation le plus précoce du sous-ensemble initial de la pluralité de bipôles au trajet de flux d'activation en tant que bipôle d'activation suivant ;
le processeur d'identification de trajet de flux (28) étant en outre configuré pour répéter les étapes consistant à :
identifier (306) un sous-ensemble subséquent de la pluralité de bipôles voisins du bipôle d'activation suivant ;
identifier (308) un bipôle d'activation le plus précoce du sous-ensemble subséquent de la pluralité de bipôles ; et
ajouter (310) le bipôle d'activation le plus précoce du sous-ensemble subséquent de la pluralité de bipôles au trajet de flux d'activation en tant que bipôle d'activation suivant ;
jusqu'à ce que le dernier bipôle d'activation soit ajouté au trajet de flux d'activation,
dans lequel le processeur d'identification de trajet de flux est configuré pour identifier le trajet de flux d'activation en identifiant de manière itérative les bipôles d'activation successifs de la pluralité de bipôles à partir des informations de synchronisation d'activation.

5. Système selon la revendication 4, comprenant en outre un processeur de cartographie configuré pour délivrer une représentation graphique du trajet de flux d'activation sur un modèle géométrique cardiaque.
